# EUROPEAN PATENT APPLICATION

(11) **EP 4 838 458 A1**
(43) Date of publication of application: **11.11.2026**
(21) Application number: 25305667.5
(22) Date of filing: 09.05.2025
(51) Int. Cl.: G06T 7/00, A61B 8/08

(54) **IMPROVED PROCESSING OF MEDICAL IMAGES**

(71) Applicant: Sonio, 75014 Paris (FR)
(72) Inventor: DEBAVELAERE, Vianney, 75014 PARIS (FR); MATEVSKI, Nikola, 75014 PARIS (FR); CORDA, Guillaume, 75014 PARIS (FR); BESSON, Rémi, 75014 PARIS (FR)
(74) Representative: Plasseraud IP

(57) **Abstract**

It is disclosed computer-implemented method comprising: obtaining at least one region of interest "ROI" defining a view in a medical image ; detecting at least one anatomical structure in the region of interest ; detecting a combination of said at least one region of interest and said at least one anatomical structure in the region of interest.

## Description

### Technical Field

This disclosure pertains to the field of medical images. In particular, the invention relates to a method for providing decision support for medical imaging.

### Background Art

Medical imaging, particularly ultrasound, is a crucial tool for detecting fetal anomalies. It allows healthcare professionals to visualize the developing fetus in real-time, providing detailed images of its anatomy and growth. During routine prenatal scans, sonographers can assess the fetus's size, position, and organ development. Specific views and measurements are used to identify potential anomalies, such as structural defects in the brain, heart, or limbs. Advanced imaging techniques, like 3D ultrasound or Doppler imaging, can further enhance the detection of abnormalities by providing more detailed views of the fetal anatomy and blood flow. Early detection of fetal anomalies through medical imaging enables timely intervention and informed decision-making for expectant parents and healthcare providers.

The accuracy of fetal anomaly detection can be significantly influenced by the expertise of the practitioners performing and interpreting the medical imaging. Experienced sonographers and radiologists are more adept at obtaining optimal images and recognizing subtle signs of anomalies. They are skilled in selecting the appropriate views and measurements necessary for a comprehensive assessment. In contrast, less experienced practitioners may struggle with image acquisition and interpretation, potentially leading to missed or incorrect diagnoses. Variability in training, familiarity with advanced imaging techniques, and the ability to identify rare or complex anomalies can all contribute to differences in detection accuracy. Consequently, the practitioner's level of expertise plays a critical role in ensuring accurate and reliable fetal anomaly detection.

There is therefore the need for a support for fetal anomalies detection in medical images.

### Summary

This disclosure improves the situation.

It is proposed a computer-implemented method comprising:
- obtaining at least one region of interest "ROI" defining a view in a medical image ;
- detecting at least one anatomical structure in the region of interest ;
- detecting a combination of said at least one region of interest and said at least one anatomical structure in the region of interest.

By "medical image", we designate a visual representation of the interior of a body for clinical analysis and medical intervention. These images are used to reveal, diagnose, or examine diseases and conditions. Medical imaging encompasses a variety of techniques and modalities that provide different types of information about the body's structures and functions. Examples of medical images comprise:
- Ultrasound Images: These are created using high-frequency sound waves to produce images of organs and structures inside the body. Ultrasound is commonly used in obstetrics to monitor fetal development, as well as in cardiology to assess heart function.
- X-ray Images: X-rays use electromagnetic radiation to capture images of the inside of the body, particularly useful for viewing bones and detecting fractures.
- Magnetic Resonance Imaging (MRI): MRI uses strong magnetic fields and radio waves to generate detailed images of organs and tissues. It is often used for brain, spine, and joint imaging.
- Computed Tomography (CT) Scans: CT scans use X-rays and computer processing to create cross-sectional images of the body, providing more detailed information than standard X-rays.
- Positron Emission Tomography (PET) Scans: PET scans use radioactive tracers to visualize metabolic processes in the body, often used in oncology to detect cancer.
- Doppler Ultrasound: A specialized form of ultrasound that evaluates blood flow through vessels, useful in assessing vascular conditions.

The medical image could be obtained in different forms, for example a DICOM image directly produced by a medical device, or a image derived from a DICOM image such as a bitmap image or a compressed image such as a JPEG image.

The region of interest may designate a part or the entirety of the medical image. The region of interest may comprise an element of interest. In one or more embodiments, the element of interest may be chosen in a predefined group specific to the medical imaging modality and/or to the medical examination. For instance, for a fetal ultrasound image, the element of interest may be an anatomical structure, for example selected in the group comprising: a brain, a vessel, an orbit, a nose, a mouth, a cavity, an abdomen etc.

In the context of medical imaging, a "view" refers to a specific orientation or angle from which an image of a particular anatomical region is captured.

For instance, for a fetal ultrasound image, the view may be a view in the group comprising the following views: a transthalamic view, a transcerebellar view, a transventricular view, an upper lip nose and nostrils view, an orbits view, a coronal view of the thorax-abdomen, a four chambers view of the heart, a left ventricular outflow tract, a right ventricular outflow tract, a three vessels view of the heart, a spine view, a transverse section of the abdomen, a bladder view, a femur diaphysis length view, a view of the two kidneys, a crown rump length (CRL) view, a nuchal translucency view, a hands view, a feet view, a profile view, a cervix length view, a 3D view and a Doppler view.

The region of interest may be manually or automatically determined. In one or more embodiments, the region of interest may be a bounded box comprising the element of interest. More specifically, in some examples, the region of interest may be the minimum bounded box comprising the element of interest.

By "An anatomical structure", we designate any distinct part of the body that occupies space and can be identified within a medical image. These structures include organs, tissues, and other bodily components that are of clinical interest. In the context of fetal ultrasound imaging, anatomical structures might include the heart, brain, vessels, limbs, and other fetal features.

A combination of a region of interest and at least one anatomical structure refers to a combination of the presence of a region of interest, and the presence, or conversely the absence of specific structures.

The integration of ROI and anatomical structures enables a comprehensive assessment of the medical image, the combination of ROI and anatomical structures supporting a more thorough evaluation of the image, for example for the need of segmenting structure for further analysis, flagging the image to healthcare professional, or detecting potential anomalies.

In another aspect, it is proposed a computing system comprising at least one processing unit and configured to execute at least a part of a method as defined here.

We designate by "processing unit" an electronic component capable of performing electronic or computer calculations for a function. A processing unit can designate any type of processor or electronic component capable of performing digital calculations. For example, a processing unit can be an integrated circuit, an ASIC (from the English acronym "Application-Specific Integrated Circuit", literally in French "integrated circuit specific to an application", a microcontroller, a microprocessor, a Digital Signal Processor (DSP), a processor, a Graphical Processing Unit (GPU). A processing unit according to the invention is not limited to a particular type of calculation architecture. For example, a processor can implement a Harvard or Von Neumann type architecture.

We designate by "Memory", or "Memory storage" a digital electronic device or digital electronic device component used to store data. Different types of memories can be used in the invention, such as read only memory (ROM), random access memory (RAM), volatile memory or flash memory. A computing device according to the invention can be equipped with one or more non-volatile memories which can be of different types such as mass memories, flash memory, read only memories or SSD memories. A computing device according to the invention may also comprise one or more random access memories such as RAM, DRAM, SRAM, DPRREAM, VRAM, eDRAM or 1T-SRAM.

In the context of the present invention, it should be understood that the terms "a," "an,", "at least one" and "one or more" are used interchangeably unless otherwise specified. Specifically, the use of the term "a" or "an" does not limit the invention to a single element but should be interpreted as including the possibility of multiple elements, unless clearly indicated otherwise. Therefore, the terms "at least one" and "one or more" also encompasses the situation where only one element is present.

In the context of the present invention, it should be understood that the expressions "at least one of ..., ... or ..." and "one or more of ..., ... or ..." are used interchangeably unless otherwise specified. Specifically, the use of the expressions "at least one of A, B or C." and "one or more of A, B or C" does not limit the invention to a single element chosen among A, B and C (A, B and C being selected by means of non limitative example only, the expressions "one or more of ... or" and "at least one of ... or" being applicable to any number of elements equal to or higher than 2) but should be interpreted as including the possibility of selecting multiple elements among A, B and C, unless clearly indicated otherwise. Therefore, the terms "at least one" and "one or more" also encompasses the situation where only one element is present. The expressions "at least one of A, B or C." and "one or more of A, B or C" encompasses all the possibilities listed below: "A or B or C or (A and B) or (A and C) or (B and C) or (A and B and C)". It shall also be noted that each of the expressions A, B and C may itself refer to more than one element, in particular if the wording "a," "at least one" or "one or more" is used

In another aspect, it is proposed a computer software comprising instructions to implement at least a part of a method as defined here when the software is executed by a processor.

In another aspect, it is proposed a computer-readable non-transient recording medium on which a software is registered to implement the method as defined here when the software is executed by a processor.

The following features, can be optionally implemented, separately or in combination one with the others:

In an embodiment:
- detecting at least one anatomical structure in the region of interest and detecting a combination of said at least one region of interest and said at least one anatomical structure in the region of interest are performed by at least one computing device;
- obtaining at least one region of interest defining a view in a medical image comprises receiving, by said at least one computing device, said medical image previously captured by a medical device physically distinct from said at least one computing device.

Stated otherwise, the processing of the medical image is performed by at least one computing device on the ground of an image previously captured by a medical device distinct from the at least one computing device.

For example, the at least one computing device can belong to a cloud service to which the image is sent, directly or indirectly, by the medical device.

This allows managing in different devices the processing of the image, and the capture of the image.

In an embodiment, the image is a 2D ultrasound image.

In an embodiment, obtaining said at least one region of interest defining a view in the medical image is performed by using a trained convolutional neural network on the medical image.

The convolutional neural network provides an efficient solution for classifying parts of an image. The training can benefit from an abundant training dataset.

In an embodiment, obtaining at least one region of interest defining a view in a medical image is performed by receiving, through a man machine interface, an input to select a view of the medical image.

The view can be selected by a user. This allows a user to select a view that is present in the image, and thus ensures that any view in the image can be selected.

In an embodiment, detecting said at least one anatomical structure in the region of interest is performed using a trained convolutional neural network on the region of interest.

The convolutional neural network provides an efficient solution for classifying parts of an image. The training can benefit from an abundant training dataset.

In an embodiment, the method further comprises:
- detecting that said medical image is to highlight based at least upon said detected combination ;
- if it is detected that said medical is to highlight, highlighting said medical image through at least one man machine interface.

Certain combinations of views and anatomical structures may be indicative of a potential anomaly. However, they may also be indicative of a medical image that has not been properly captured.

A "potential anomaly" refers to an indication or sign that suggests the presence of an irregularity or deviation from the expected norm within a medical image. It is a preliminary finding that may warrant further investigation to determine whether an actual abnormality exists. In the context of fetal ultrasound imaging, a potential anomaly could involve unusual features or measurements in anatomical structures, such as the placenta, that might indicate developmental issues or other medical concerns.

A medical image to highlight is a medical image that requires a specific attention for the practitioners. A medical image to highlight may for example be a medical image where a conditions indicative of a potential anomaly has been detected. Thus, such an image needs to highlight to the practitioner so that the practitioner can examine the image with specific care to determine whether it represents an anomaly or not.

By " 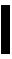 highlighting said medical image", we designate rendering elements associated with the medical image to the healthcare professional through a man-machine interface when it is detected that the medical image is to highlight. Highlighting a medical image serves as a prompt for the practitioner to further investigate the medical image. It can be performed in one or more forms, such as:
- **Visual Display:** The medical image can be highlighted on the screen of a device being used, such as a tablet or computer, for example by:
   - representing the medical image in a way that renders it more visible than other medical images, for example by one or more of:
      - displaying the highlighted image in a specific color ;
      - framing the highlighted medical image in a specific, for example flashy color. For example, highlighted images can appear in a purple frame ;
      - displaying the highlighted medical image in a larger scale than other images
      - displaying a text indicating that the image is highlighted onto or near to the image. The text can simply indicate that the image is highlighted / needs a specific care, or indicate reasons why the image is highlighted, for example a potential anomaly that has been detected in the image.
- **Auditory Signal:** An accompanying sound, such as a beep or alarm when the highlighted image is displayed , can be used to ensure the practitioner is aware that a highlighted image is displayed even if they are not looking directly at the screen.
- **Integration with User Interface:** The indication that the image is highlighted can be integrated into an existing application, providing contextual information alongside the medical images and detected views.

The highlight of the image, and, when applicable, the indication of an associated potential anomaly, is designed to be non-diagnostic, meaning it does not provide a definitive diagnosis but rather indicates that further examination is warranted. This ensures that healthcare professionals are promptly informed of potential issues.

The integration of ROI and anatomical structures enables a comprehensive assessment of the medical image, considering both the spatial relationships and morphological characteristics of the structures. This holistic view supports a more thorough evaluation of potential anomalies.

The method thus allows for an effective detection of a potential issue, and alerting of practitioners.

In an embodiment, the method comprises highlighting said medical image upon the occurrence of at least one condition relative to the detection that said medical image is to highlight, and at least one of:
- the detection that in at least one further medical image captured by the same medical device than said medical image is to highlight ;
- the detection of a presence or absence of at least one further condition of highlight in said medical image, or in at least one further medical image captured by the same medical device than said medical image ;
- the detection of a presence or absence of at least one further anatomical structure in said medical image, or in at least one further medical image captured by the same medical device than said medical image.

By "condition of highlight", we designate a specific combination of elements in the medical image that triggers the detection that the image is to highlight.

Stated otherwise, highlighting a medical image, that can be called "aggregated alert" in this case, is not performed upon the detection of a single image to highlight (for example a single potential anomaly in a single image), but on more complex rules that may comprise:
- a repetition of a same condition for highlighting images, for example the same potential anomaly, in different and temporally close images captured by the same medical device;
- a combination of conditions for highlighting images, for example a combination of potential anomalies, in the same image, or temporally close images captured by the same medical device ; and / or
- a combination of a detection that an image is to highlight (for example at least one potential anomaly), and the presence or absence of certain anatomical structures.

This allows defining more complex alert rules, to highlight images in a more accurate way.

In an embodiment, the method comprises detecting that said medical image is to highlight if:
- said view is a Left Ventricular Outflow Tract "LVOT" ;
- said at least one anatomical structure comprises at least one of:
   o a left ventricle and a pulmonary artery trunk ;
   o a left ventricle and a pulmonary artery bifurcation ;
   o a pulmonary artery trunk and ascending aorta and a left ventricle ;
   o a right ventricle and an ascending aorta ;
   o a pulmonary artery bifurcation but no ascending aorta.

The conditions listed above are representative of a potential malposition of great vessels. This therefore allows highlighting the medical image to allow the practitioner to check for a potential malposition of great vessels.

In an embodiment, the method comprises detecting that said medical image is to highlight if:
- said view is a transthalamic view ;
- said at least one anatomical structure does not comprise a cavum septum pellucidum.

The conditions listed above are representative of a potential absence of cavum septum pellucidum. This therefore allows highlighting the medical image to allow the practitioner to check for a potential absence of cavum septum pellucidum.

In an embodiment, the method comprises comprising detecting that said medical image is to highlight if:
- said view is a corpus callosum view ;
- said at least one anatomical structure does not comprise a corpus callosum.

The conditions listed above are representative of a potential absence of corpus callosum This therefore allows highlighting the medical image to allow the practitioner to check for a potential absence of corpus callosum.

In an embodiment, detecting the presence or absence of at least one potential anomaly comprises, upon a condition relative to said combination :
performing a segmentation of said at least one anatomical structure in the region of interest of the image..

By "segmentation of an anatomical structure", we refer to the process of partitioning a distinct region of a medical image, which corresponds to a specific anatomical structure of interest.

Segmentation allows for the isolation of specific anatomical structures within the region of interest, enabling a more detailed examination of their shape, size, and position. The combination of ROI and anatomical structures provides a good criterion to determine when a segmentation of anatomical structures may be beneficial.

In an embodiment said performing the segmentation comprises:
- retrieving a segmentation model associated to said view ;
- inputting to said segmentation model, on separate channels :
   o at least one bounding box corresponding respectively to each of said at least one anatomical structure ;
   o said medical image ;
- receiving, as output from the segmentation model, at least one structure segmentation mask corresponding respectively to each of said at least one anatomical structure.

Using a model specifically trained for the view provides an accurate segmentation of the structures of the view. The method thus provides an overall improved methods for segmenting anatomical structures in a medical image.

Providing as input to the segmentation model the complete image in addition to the bounding boxes of the structures, allows providing an even more accurate result, because some data from the structures that would not be properly segmented in a bounding box can be retrieved from the complete image.

The additional steps thus provide an accurate segmentation method for segmenting the anatomical structures.

In an embodiment:
- the segmentation model comprises a fully convolutional network that comprises one input channel for the medical image, and one input channel for each of the anatomical structures associated with the view ;
- said inputting comprises:
   o converting said medical image, and each of said at least one bounding box into grayscale images ;
   o Inputting said grayscale images to said segmentation model.

The convolutional network specifically trained for the view is particularly efficient to generate segmentation masks based on grayscale images.

In an embodiment, the method further comprises:
- performing an analysis of the at least one segmented structure
- detecting that said medical image is to highlight based upon the output of said determination of anatomical structures and said analysis of said at least one segmented structure..

By "at least one segmented structure", we refer to the distinct region of the medical image that is outputted by the segmentation process, and that is deemed to contain all the pixels of the medical image that form the anatomical structure.

Analyzing the at least one segmented structure involves evaluating the segmented anatomical structures to identify any irregularities or deviations from the norm that may indicate that the image is to highlight, for example by indicating a potential anomaly.

The list of the detected anatomical structures provides an insight that is complementary to the segmentation of the anatomical structures.

Segmentation allows for the isolation of specific anatomical structures within the region of interest, enabling a more detailed examination of their shape, size, and position. The analysis of segmentation of anatomical structures therefore improves the accuracy of the detection of potential anomalies. The analysis of segmentation also provides the advantage of requiring a limited number of training images to detect potential anomalies.

Thus, an accurate detection of images to highlight and potential anomalies can be performed, even if a limited number of abnormal images are known for training, which may notably be the case for rare anomalies.

In an embodiment:
- said condition relative to the combination is validated if:
   ∘ said view is a 3 vessels view, and
   ∘ said at least one anatomical structure comprises a pulmonary artery, an ascending aorta and a superior vena cava ;
- said analysis of the at least one segmented structure comprises a computation of a width of the pulmonary artery, a width of the ascending aorta and a width of the superior vena cava.

The width of an anatomical structure refers to the measurement of the structure's extent across its shortest dimension within a medical image. This measurement is typically taken perpendicular to the longest axis of the structure. In the context of medical imaging, determining the width of an anatomical structure can provide valuable information about its size and shape, which is important for assessing normalcy or identifying potential anomalies.

For example, in a cross-sectional image of a blood vessel, the width would be the distance across the vessel's lumen, perpendicular to its length. Similarly, in a cardiac ultrasound image, the width of a heart chamber might be measured from one side of the chamber to the other, perpendicular to the chamber's length.

By means of non limitative example, the width of a structure can be calculated from the segmentation mask of the structure by:
- calculating the longest axis of the structure, which is typically the major axis of the best-fit ellipse that can be drawn around the structure. This axis represents the longest dimension of the structure ;
- measuring the width as the shortest dimension perpendicular to the longest axis. This can be done by finding the minimum distance between parallel lines that are tangent to the structure's contour on either side of the longest axis.

The width can either be calculated in number of pixels, or converted to real-world units.

This allows automatically detecting a potential anomaly in case of inconsistent widths of the 3 vessels, and highlighting the medical to the practitioner accordingly.

In an embodiment, the method comprises detecting that said medical image is to highlight, if a ratio between the width of the pulmonary artery, and the width of the aorta is above a predefined threshold.

A ratio between a first element and a second element is a quantitative relationship that expresses how many times one element contains or is contained within the other. It is calculated by dividing the value of the first element by the value of the second element. Mathematically, if *A* is the first element and *B* is the second element, a ratio between *A* and *B* is expressed as: $Ratio = \frac{A}{B}$

The conditions listed above are representative of a potential asymmetry of the great artery. This therefore allows highlighting the medical image to allow the practitioner to check for a potential asymmetry of the great artery.

In an embodiment, the method comprises detecting that said medical image is to highlight, if a ratio between the width of the aorta, and the width of the superior vena cava is above a predefined threshold.

The conditions listed above are representative of a potential hypoplasia of the ascending aorta. This therefore allows highlighting the medical image to allow the practitioner to check for a potential hypoplasia of the ascending aorta.

In an embodiment:
- said condition relative to the combination is validated if:
   o said view is a 3 vessels view, and
   o said at least one anatomical structure comprises a pulmonary artery, an ascending aorta and a superior vena cava ;
- said analysis of the at least one segmented structure comprises a verification of the alignment of the pulmonary artery, the ascending aorta and the superior vena cava;
- said method comprises detecting that said medical image is to highlight if said verification is not fulfilled.

The conditions listed above are representative of a potential misalignment of the 3 vessels. This therefore allows highlighting the medical image to allow the practitioner to check for a potential misalignment of the 3 vessels.

In an embodiment,
- said condition relative to the combination is validated if:
   o said view is a 4 chamber view, and
   o said at least one anatomical structure comprises a left ventricle, a right ventricle, a left atrium, and a right atrium ;
- said analysis of the at least one segmented structure comprises a computation of a width of the left ventricle, a width of the right ventricle, a width of the left atrium, and a width of the right atrium;
- said method comprises detecting that said medical image is to highlight if:
   ∘ a ratio between the width of left atrium and the width of the right atrium is not in a first predefined range ; or
   ∘ a ratio between the width of left ventricle and the width of the right ventricle is not in a second predefined range.

The conditions listed above are representative of a potential disequilibrium of the chambers. This therefore allows highlighting the medical image to allow the practitioner to check for a potential disequilibrium of the chambers.

In an embodiment,
- said condition relative to the combination is validated if:
   ∘ said view is a 4 chamber view, and
   ∘ said at least one anatomical structure comprises a left ventricle, a right ventricle, a left atrium, a right atrium and a thorax contour ;
- said analysis of the at least one segmented structure comprises :
   ∘ a determination a surface of the heart in the medical image as the sum of the areas of segmentation masks of the left ventricle, the right ventricle, the left atrium, and the right atrium ;
   ∘ a determination of a surface of the abdomen in the medical image as the area of the segmentation mask of the thorax contour ;
   ∘ a calculation of a ratio between the surface of the heart and the surface of the abdomen ;
said method comprises detecting that said medical image is to highlight if said ratio is above a predefined threshold

The area of a segmentation mask refers to the number of pixels that are classified as part of a particular object or region within an image. In the context of medical imaging, a segmentation mask is typically a binary image where pixels belonging to the object of interest (e.g., an anatomical structure) are marked with a value of 1 (or true), and all other pixels are marked with a value of 0 (or false).

The area of the segmentation mask can thus be determined by counting the pixels that belong to the mask. The area of the segmentation mask can either be kept as a number of pixels, or converted to a physical area.

A ratio above the predefined threshold indicates that the heart is abnormally large compared to the abdomen.

The conditions listed above are representative of a potential abnormally large heart. This therefore allows highlighting the medical image to allow the practitioner to check for a potential abnormally large heart.

In an embodiment,
- said condition relative to the combination is validated if:
   o said view is a 4 chamber view, and
   ∘ said at least one anatomical structure comprises an interventricular septum, a spine, a left ventricle, a right ventricle, a left atrium, a right atrium and a thorax contour ;
- said analysis of the at least one segmented structure comprises :
   ∘ a determination of a first line (Lin7.1) going through the septum ;
   ∘ a determination of a second line (Lin7.2) that contains the center of a bounding box enclosing the spin and divides the thorax contour into two parts of equal surface ;
   ∘ a calculation of an angle (Ang7) between the first line and the second line ;
said method comprises detecting that said medical image is to highlight if said angle is not in a predefined range.

The conditions listed above are representative of a potential deviation of the heart axis. This therefore allows highlighting the medical image to allow the practitioner to check for a potential deviation of the heart axis.

### Brief Description of Drawings

Other features, details and advantages will be shown in the following detailed description and on the figures, on which:
**Fig. 1**

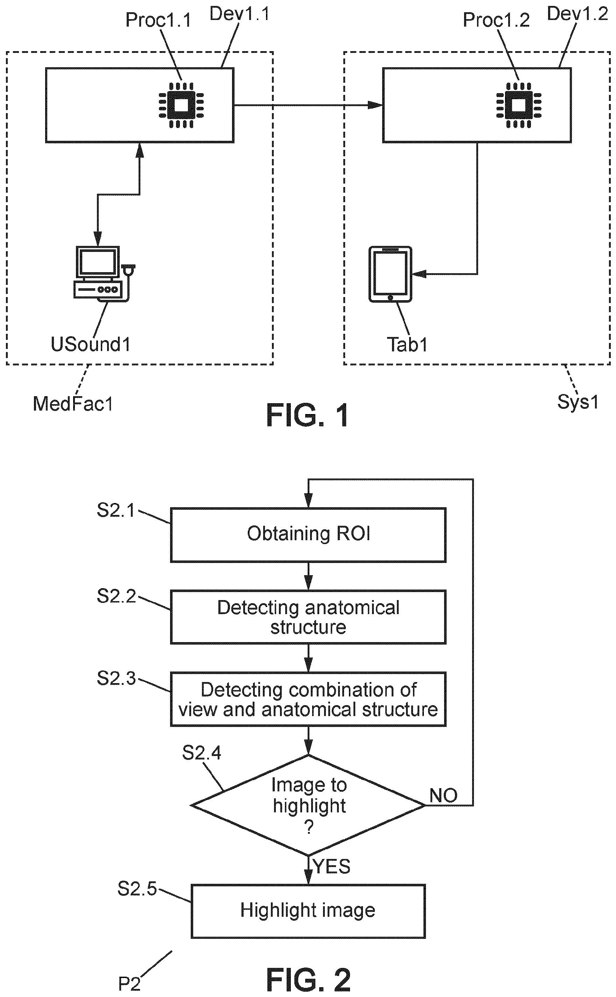

   [Fig. 1] is an example of a computing system according to an embodiment.
**Fig. 2**
   [Fig. 2] is an example of a computer-implemented method according to an embodiment.
**Fig. 3**

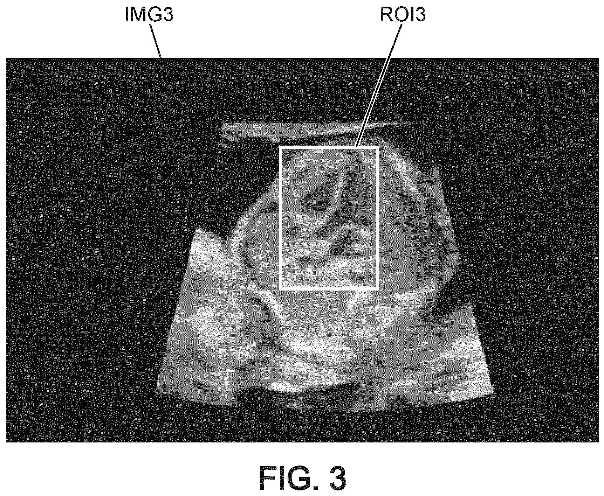

   [Fig. 3] is an example of detection in a medical image of a ROI defining a LVOT according to an embodiment.
**Fig. 4**

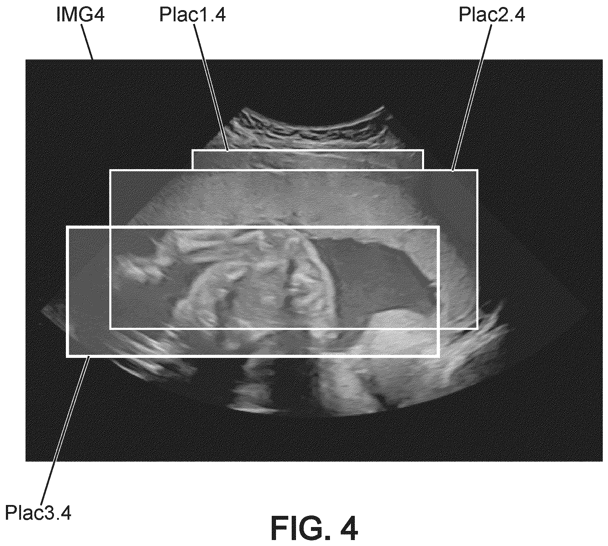

   [Fig. 4] is an example of labelling of a medical image for the training of a deep learning model to detect anatomical structures in a medical image according to an embodiment.
**Fig. 5**

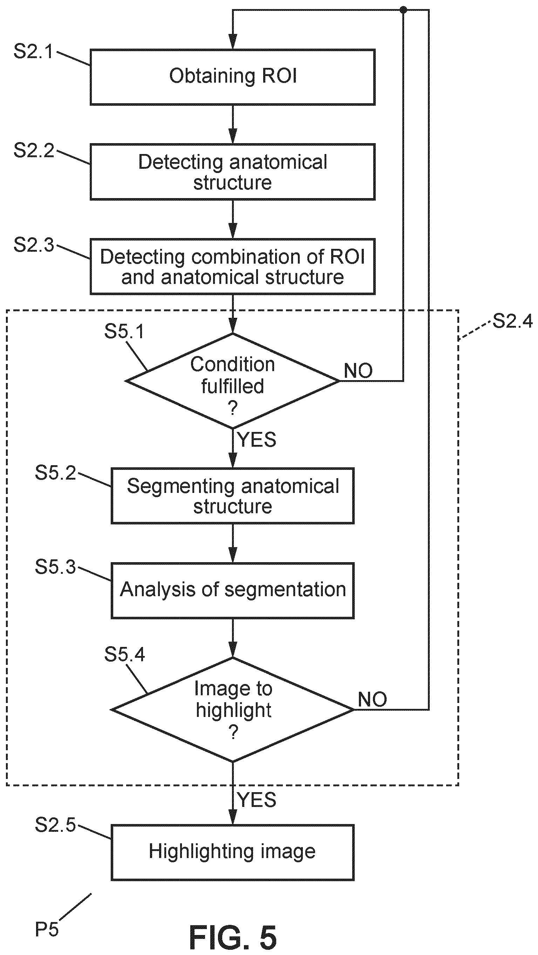

   [Fig. 5] is an example of a computer-implemented method comprising a segmentation of at least one anatomical structure according to an embodiment.
**Fig. 6**

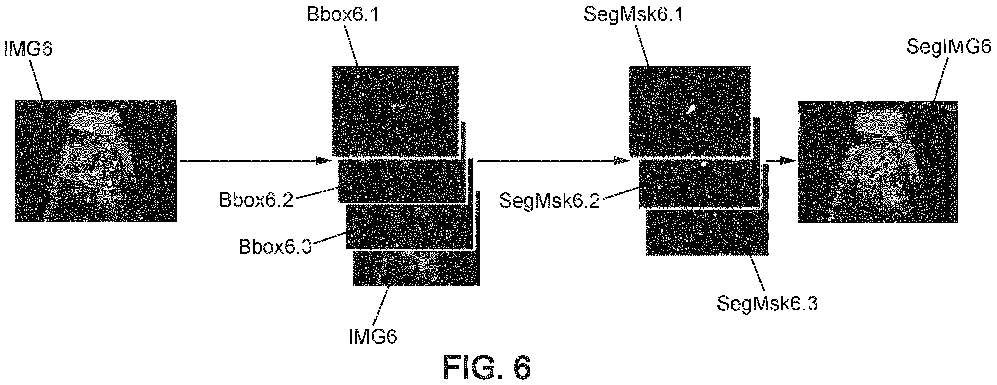

   [Fig. 6] is an example of detection and segmentation of anatomical structures using a segmentation model associated with a specific view. according to an embodiment.
**Fig. 7**

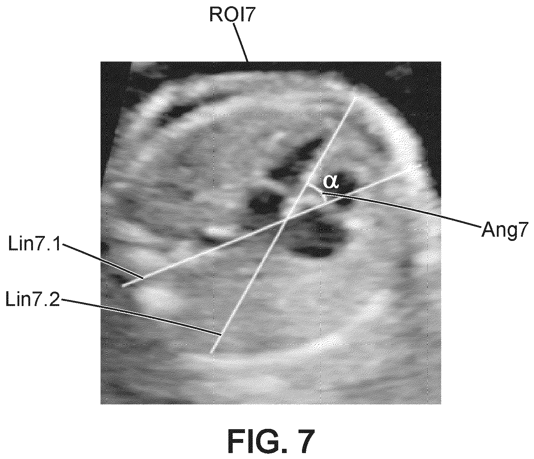

   [Fig. 7] is an example of detection of potential deviation of the heart axis according to an embodiment.

### Description of Embodiments

It is now referred to figure 1.

Figure 1 is an example of a computing system according to an embodiment.

Figure 1 illustrates a computing system, Sys1, designed for processing ultrasound images.

The computing system Sys1 comprises a second computing device Dev1.2, and a tablet Tab1, and is configured to process medical images received from a medical facility MedFac1. the medical facility MedFac1 comprises an ultrasound device Usound1, and a first processing device Dev1.1. It is worth noting that, although the second computing device Dev1.2, and the tablet Tab1 belong to the same computing system Sys1 and be provided by the same service provider, they are not necessarily located in the same place. In particular, the tablet Tab1 can be located in the same room than the ultrasound device USound1.

The ultrasound device USound1 is responsible for generating DICOM images, for example when a practitioner performs fetal ultrasound analysis.

The DICOM images are then sent to the first computing device Dev1.1. This device is equipped with a first processing unit Proc1.1. The primary function of Proc1.1 is to process the incoming DICOM images by compressing them into thumbnail images, typically in JPEG format, and extracting metadata. These thumbnail images, are then queued for transmission to the second computing device Dev2.1.

The second computing device Dev2.1, containing a second processing unit Proc2.1, performs image analysis. Once the images are received by the second computing device Dev2.1, Proc2.1 analyzes them. For example, the second computing device Dev2.1 can implement all or a part of a method according as represented in the figures 2 and 3.

The results of the analysis, along with the images, are then transmitted to a tablet Tab1. The tablet runs a web app, which displays the images and the detected views. This allows the practitioner to review the images and, if necessary, manually assign views to the images if the automatic detection does not yield satisfactory results.

Thus, the practitioner has access to both the ultrasound device USound1, and tablet Tab1. The tablet Tab1 shows relevant information in parallel to the ultrasound device USound1, thereby helping the practitioner to perform ultrasound fetal examination.

The first computing device Dev1.1, and second computing device Dev2.1 can be located in different places. For example, the first computing device Dev1.1 may be a local server of a hospital where the ultrasound device USound1 is located, while the second computing device Dev2.1 can be a distant server, thereby rendering a cloud-based analysis.

This configuration is however provided by means of example only of systems in an embodiment. For example, the first computing device Dev1.1 and the second computing device Dev2.1 can be located in various configurations to suit different operational needs and infrastructure setups:
- **Local Configuration:**
   o **Dev1.1:** This device can be situated within the hospital or medical facility where the ultrasound device USound1 is located. It can serve as a local server or workstation that processes images on-site, ensuring quick access and minimal latency.
   o **Dev2.1:** This device can also be located within the same facility, perhaps in a dedicated IT room or data center, to maintain a fully localized system. This setup can be beneficial for facilities with robust internal networks and IT support.
- **Distributed Configuration:**
   o **Dev1.1:** Positioned at the hospital or clinic, acting as an edge device that performs initial processing and compression of images before sending them to a remote location.
   o **Dev2.1:** Located off-site, such as in a centralized data center or cloud service provider's facility. This setup allows for scalable processing power and storage, leveraging cloud computing resources for intensive image analysis tasks.
- **Hybrid Configuration:**
   o **Dev1.1:** Located on-site at the medical facility, providing immediate processing capabilities and serving as a buffer for data before it is transmitted.
   o **Dev2.1:** Situated in a regional data center that serves multiple facilities, offering a balance between local processing and centralized analysis. This configuration can optimize resource use and provide redundancy.
- **Cloud-Based Configuration:**
   o **Dev1.1:** Functions as a lightweight client device, possibly even integrated into the ultrasound machine itself, focusing on data transmission rather than processing.
   ∘ **Dev2.1:** Fully cloud-based, with all processing and analysis conducted in the cloud. This setup minimizes on-site hardware requirements and allows for flexible scaling of computational resources.

Each configuration offers distinct advantages and can be selected based on factors such as network infrastructure, data security requirements, and the scale of operations.

In the context of the system architecture depicted in FIG. 1, it is important to note that the configuration can include more than one computing device and more than one processing unit. For example:
- **Computing Devices (Dev1.1, Dev2.1):** While the figure illustrates a single first computing device (Dev1.1) and a single second computing device (Dev2.1), the system can be expanded to include multiple computing devices. This allows for distributed processing, where different devices can handle various tasks such as image compression, metadata extraction, and image analysis. Multiple computing devices can be strategically placed in different locations, such as within the hospital or in remote data centers, to optimize processing efficiency and resource allocation.
- **Processing Units (Proc1.1, Proc2.1):** Similarly, each computing device can be equipped with more than one processing unit. These processing units can work in parallel to enhance computational power and speed up the processing of ultrasound images. For instance, additional processing units can be used to handle more complex image analysis tasks or to support redundancy and fault tolerance in the system.

The flexibility to incorporate multiple computing devices and processing units allows the system to scale according to the needs of the medical facility.

In the system architecture depicted in FIG. 1, while a tablet (Tab1) is illustrated as the user device for displaying ultrasound images and analysis results, it is important to note that other user devices can also be utilized. These devices include:
- **Desktop Computers:** A desktop computer can be used to access a web app, providing a larger screen for detailed image analysis and review.
- **Laptops:** Laptops offer portability and flexibility, allowing practitioners to access the system from different locations within the medical facility.
- **Smartphones:** Although smaller in screen size, smartphones can be used for quick access to images and results, especially in situations where mobility is important.
- **Smart Displays or Monitors:** These can be integrated into the medical facility's infrastructure to provide a dedicated display for continuous monitoring and review of ultrasound images.

The choice of user device can be tailored to the specific needs and preferences of the healthcare professionals, ensuring that they have convenient and efficient access to the system's capabilities.

This system architecture, featuring distributed processing and cloud-based analysis, enhances the efficiency and accuracy of ultrasound image processing. The design provides real-time feedback and decision support for healthcare professionals. It is however worth noting that the processing performed by the computing device Dev2.1, and the elements shown by the tablet Tab1 are only designed to provide relevant information to the practitioner, but are not a diagnosis. The diagnosis remained performed by the practitioner on the ground of the images directly provided by the ultrasound device USound1, the images and information displayed by the tablet Tab1, and analyzed by the computing device Dev2.1 being only aimed to highlight some relevant information to the practitioner.

The communication of compressed images, such as for example JPEG images, rather than DICOM images, from the first second computing device Dev1.1 to the second computing device Dev2.1 also allows using cloud-based services with a limited latency, because the compressed images have a drastically lower size compared to DICOM images.

Although the system Sys1 is exemplified with an ultrasound device USound1, the system architecture provided in figure 1 is provided by means of non-limitative example only of a medical system that can implement a method in an embodiment of the invention. In particular, the system architecture shown in figure 1 could be applied to medical devices belonging to other types of medical devices than an ultrasound device.

It is also worth noting that the system Sys1 is provided only by means of example of a computing system in an embodiment of the invention. In other embodiments, other architectures may be used. For example, the capture and processing of medical images, as well as the rendering to the user may be performed in the same device.

It is now referred to figure 2.

The figure 2 shows a flowchart illustrating a computer-implemented method P2 for providing decision support in medical imaging, specifically aimed at highlighting medical images that require specific care for example to detect potential anomalies in medical images. This method is designed to assist practitioners by highlighting areas of concern that may warrant further investigation.

The method P2 will be generally discussed with reference of fetal ultrasound images, and fetal anomalies. The invention is however not restricted to the analysis of fetal ultrasound images, and could for example be applied to other types of medical imaging scenarios where anomaly could be detected, such as for example:
- Cardiac Imaging, in order to detect anomalies in adult or pediatric heart structures using echocardiography or MRI;
- Oncological Imaging, in order to identify potential tumors or irregularities in tissues using CT scans, MRI, or PET scans;
- Neurological Imaging, in order to detect abnormalities in brain structures through MRI or CT scans;
- Musculoskeletal Imaging, in order to identify fractures, tears, or other anomalies in bones and soft tissues using X-rays, MRI, or ultrasound;
- Abdominal Imaging, in order to detect issues in organs such as the liver, kidneys, or pancreas using ultrasound, CT, or MRI;
- Vascular Imaging, in order to identify blockages or irregularities in blood vessels using Doppler ultrasound or angiograph ;
- Etc.

The method P2, as described in figure 2, can for example be implemented, totally or in part, by the second computing device Dev2.1 in FIG. 1. More generally, the method P2 can be implemented by a computing system comprising at least one computing device to analyze the medical image, and a Man Machine Interface to render alerts, and optionally receive inputs from a user.

The method P2 begins with step S2.1, which involves obtaining at least one Region Of Interest (also abbreviated "ROI") within a medical image, which can be denoted "IMG".

The medical image can be for example obtained, directly or indirectly from a medical device such as the ultrasound device USound1, as depicted in figure 1. The ultrasound device generates DICOM images during the ultrasound examination. For example, the medical images captured by a medical device can be processed as early as possible after being captured. Obtaining the medical image can thus amount to receiving the medical image at the second computing device Dev1.2.

In the example of figure 1; the images are sent, as early as they are captured by the ultrasound device USound1, to the first computing device Dev1.1, where they are processed by the first processing unit Proc1.1. This processing includes compressing the images into compressed format, preparing them for further analysis by the second computing device Dev2.1. In the context of figure 1, the medical image that is analyzed by the method P2 would therefore be the compressed image received by the second computing device Dev2.1. This is however provided by means of example only. In other embodiments of the invention, the image that is analyzed could for example be the DICOM image.

The medical image could also be a previously stored image.

The region of interest (ROI) in a medical image can designate a part or the entirety of the image that is relevant for analysis. It may include specific anatomical features or areas that are of clinical interest. In the context of fetal ultrasound imaging, the ROI might comprise elements such as the brain, heart, vessels, or other fetal structures. The ROI can be determined manually by a practitioner or automatically using image processing techniques, such as a convolutional neural network, which can efficiently classify and highlight parts of an image for further examination. The ROIs can be obtained at step S2.1 in different ways from the medical image.

In an embodiment of the invention, a ROI and the corresponding view is entered by the practitioner, for example through a tactile screen of the tablet Tab1. For example, the medical image can first be displayed in the tablet Tab1, then the practitioner can select the ROI directly on the image, and select the associated view. These elements can then be sent to the second computing device Dev2.1 for further analysis.

More generally the at least one ROI can be obtained by receiving an input through a man machine interface. The man machine interface can for example simultaneously display the medical image and receive an input, for example from a practitioner, that selected at least one ROI in the image. For example, a tactile screen can display the medical image and simultaneously let a practitioner select the at least one ROI, and the view defined by the at least one ROI.

In another embodiment of the invention the invention, ROIs can be obtained at step S2.1 by automatically analyzing the medical image. For example, ROIs can be obtained using deep learning models, such as for example a previously trained convolutional neural network (CNN). The model that detect the ROI representing views will be for the sake of simplicity referred as DETECT_VIEW.

In an embodiment, the Yolov5 model, which is a real-time convolutional neural network (CNN) described by Khanam, R., & Hussain, M. (2024). What is YOLOv5: A deep look into the internal features of the popular object detector. arXiv preprint arXiv2407.20892.*,* is used as DETECT_VIEW model with the following characteristics:
- Data augmentation has been applied: HSV augmentation, affine transformation, and flip left-right.
- The network was pre-trained using the COCO (Common Objects in Context) dataset.
- The network takes as input a 640x640 image. The images are resized before passing through the network.
- For each region of interest, a detection threshold needs to be defined. A view is detected if its associated region of interest is returned by the network with a confidence higher than the associated threshold.

The model can be trained using labels provided by a large number of practitioners on a training database of medical images. In practice, the labels correspond to associating a medical image of a training set to one or more ROI and a corresponding view. Thus, the trained model DETECT_VIEW is then able, in the step S2.1 to detect, from a previously unknown medical image, at least one ROI, each ROI being associated with a specific view.

The model DETECT_VIEW may for example be executed by the processing unit Proc2.1.

The step S2.1 thus defines the specific area of the image that will be analyzed for potential anomalies.

It is now referred to figure 3.

FIG. 3 shows an example of detection of a region of interest ROI3 defining a Left Ventricular Outflow Tract "LVOT" view in a medical image IMG3. This figure illustrates the output of the step S2.1.

The medical image IMG3 represents a common 2D ultrasound image utilized in prenatal examinations to assess fetal development. Within this image, the region of interest ROI3 is highlighted, indicating the area where the LVOT view is detected. The LVOT view holds importance in fetal echocardiography as it provides a detailed view of the heart's outflow tract, which plays a critical role in evaluating the heart's structure and function.

The detection of ROI3 within IMG3 demonstrates the system's capability to automatically identify and focus on important anatomical regions.

It is now referred back to figure 2.

Following the identification of the ROI, step S2.2 involves detecting at least one anatomical structure within the ROI. This detection is typically performed using advanced image analysis techniques, such as a convolutional neural network, which can be trained to recognize specific anatomical features within the ROI. The step S2.2 can for example be implemented by the second computing device Dev2.1 represented in the figure 2, and thus be executed by the processing unit Proc2.1.

In an embodiment of the invention, anatomical structures can be obtained at step S2.2 using deep learning models, such as for example a convolutional neural network (CNN). The model that detect the ROI representing views will be for the sake of simplicity referred as DETECT_STRUCTURE.

In an embodiment, the DETECT_STRUCTURE model is, as the DETECT_VIEW model discussed with reference to step S2.1, the Yolov5 model. Of course, the DETECT_STRUCTURE model is trained with different training set than the DETECT_VIEW model. For example, the DETECT_STRUCTURE can be trained with the following characteristics:
- Data augmentation has been applied: HSV augmentation, affine transformation, and flip left-right.
- The network was pre-trained using the COCO (Common Objects in Context) dataset.
- The network takes as input a 640x640 image. The images are resized before passing through the network.
- For each anatomical structure, a detection threshold needs to be defined. A view is detected if its associated region of interest is returned by the network with a confidence higher than the associated threshold. A F1 score can be used.

The DETECT_STRUCTURE model can be trained using labels provided by a large number of practitioners on a training database of medical images. In practice, the labels correspond to associating a medical image of a training set to one or more anatomical structures that can be for example each represented by a bounding box in the medical image. Thus, the trained model DETECT_STRUCTURE is then able, in the step S2.2 to detect, from a previously unknown medical image, at least one anatomical structure. As will be explained in more detail below, each view can be associated with certain anatomical structures, and the model DETECT_STRUCTURE can be used in step S2.2 to detect only the anatomical structures associated with a view detected in step S2.1.

It is now referred to figure 4.

Figure 4 is an example of labelling of a medical image for the training of a deep learning model to detect anatomical structures in a medical image according to an embodiment

Figure 4 presents an example of labeling a medical image IMG4 for training a deep learning model to detect anatomical structures. In the example of figure 4, the image IMG4 is an image of the placenta.

The medical image IMG4 serves as the base image upon which the labeling is performed. This image provides the visual context needed for identifying the anatomical structures of interest.

In the example of figure 4, a labeler (which can be a practitioner) identified a first, a second and a third structures of the placenta, respectively noted Plac1.4, Plac2.4 and Plac3.4. Each of the structure is identified by a smallest rectangle that encompasses the structure.

The image IMG4 can thus be used to trained a deep learning model (for example a CNN such as for example Yolov5) to identify the third structures Plac1.4, Plac2.4 and Plac3.4 in a medical image of the placenta.

The figure 4 is provided by means of non limitative example only of annotation of structures, and other annotations may be used, in particular for other views than placenta.

It is now referred back to figure 2.

The method P2 further comprises a step S2.3 of detecting a combination of said at least one region of interest and said at least one anatomical structure in the region of interest.

Such a combination provides a good insight regarding the views, for example with respect to potential anomalies. In particular as will be explained below, the combination provides good insights regarding the detection of potential anomalies, the display of information to a practitioner, and/or the segmentation of structures.

Although the method P2 is described in an embodiment relative to the detection of potential anomalies, the invention is not restricted to this example, and the detection of the combination can be used for other purposes, for example the segmentation of structures and/or display of information to a practitioner.

The method P2 further comprises a step S2.4 of detecting that said medical image is to highlight .

This detection of detecting that the medical image is to highlight is based at least on the combination of the identified ROI and the detected anatomical structures detected at step S2.3.

Stated otherwise, an image may be determined to highlight depending at least upon the detection of a specific ROI (and thus view), associated to the presence (or conversely the absence) of at least one specific anatomical structure. The medical image may for example be highlighted because the detection indicates a potential anomaly.

Many examples of such predictions will be provided below.

A detection that an image is to highlight, for example through the detection of potential anomaly based on ROI and anatomical structures enables a comprehensive assessment of the medical image, considering both the spatial relationships and morphological characteristics of the structures. This holistic view supports a more thorough evaluation of potential anomalies. The step S2.4, based upon step S2.3, therefore provides an efficient detection of images to highlight, notably in view of potential anomalies detected in the images.

If it is not detected that the image is to highlight, the method can return to step S2.1 to images to highlight on the ground of further ROIs and/or further images.

If it is detected that the image is to highlight, a step S2.5 of highlighting the medical image through at least one man-machine interface. For example, the image can be displayed to be particularly visible (by displaying a colored frame around the image, displaying the image in a specific color, etc.), an alert can be displayed in superimposition to the medical image, etc. The rendering of the image and the highlight can be performed on the tablet Tab1 or another user device, .

When an image is highlighted, various types of notifications can be sent to the practitioner. For example, the one or more of the following highlights/alerts can be rendered to the practitioner:
- Visual Highlight: Displayed in addition to the image on the screen of the device being used, such as a tablet or computer. This could be in the form of a colored frame, pop-up message, highlighted text, or a color-coded indicator that draws attention to the image.
- Auditory Highlight: Sound notifications, such as beeps or alarms, can accompany or replace visual highlights when the highlighted image is displaeyd to ensure that the practitioner is aware that an image is highlighted even if they are not looking directly at the screen.

Highlighting the image may comprise displaying an alert that for example shows the name of a potential anomaly detected in the image, and can be displayed on top of or near the medical image, for example through the tablet Tab1.

As noted above, alerts can be raised on the ground of each image. Then, an alert can be rendered when detected on the ground of each image. More generally, each image can be determined to highlight or not.

Conversely, aggregated alerts can be raised instead of alerts that are based on a single image and detection of anomalies. An aggregated alert is therefore an alert that is based, not on a single image and a single anomaly, but on a combination of alerts/potential anomalies and/or images. The aggregation is done differently depending on the nature of the anomaly, using the rationale that for some anomalies, abnormal images supersede normal images while it can be the reversed for other anomalies. More generally, an image can be highlighted, not when a condition for highlight is detected in a single image, but different conditions for highlight are detected in different or the same image. The numbered paragraph below will discuss the raising of an aggregated alert, but the disclosure of the paragraph below can more generally apply to the highlight of one or more images on the ground of aggregated conditions.

The aggregation can be performed in different ways.

For example, an aggregated alert can be rendered if at least two alert are raised in a short time frame (e.g when the time duration between the two alerts is below a threshold).

More generally, an aggregated alert can be raised upon a detection of the presence of a potential anomaly in a number of medical images higher than a predefined threshold in a group of successive medical images captured by the same medical device.

By "a group of successive medical images captured by the same medical device", we designate a series of medical images captured by a same device at successive time stamps. Such a group may have a predefined size, for example 10 images. By means of example, such a group may thus be a series of 10 images captures by a same device at n (for example 10) successive time stamps *tᵢ, tᵢ₊₁, tᵢ₊₂ ... tᵢ₊ₙ.*

Thus, an aggregated alert can be detected when a same potential anomaly is detected in a plurality of different images. For example, if, in a group of successive images, the potential anomaly is detected in a predefined number of images. The predefined number may be set to cover the whole group, or a predefined fraction of the group. For example, an aggregated alert may be raised if 5, 6 or even 10 images in a group of 10 successive images captured by the same medical device raise the same alert.

More complex rules of alert aggregation can be foreseen.

For example, when a single image has been captured, and an alert raised in the image:
- an aggregated alert can be raised when the alert is relative to certain types/combinations of alerts (for example alerts related to stomach or umbilical vein (the term "umbilical vein" being also referred to by the acronym "UV"). The type of alerts that automatically generate an aggregated alert on the ground of a single image can be for example the types of alerts/potential anomalies that are the most critical, or that are usually associated to the highest confidence that an anomaly is likely present ;
- otherwise, if an alert is raised on the ground of a single captured image, but this image does not relate to the types of alerts that automatically raise an aggregated alert, a notification to recommend to capture further images can be raised, in order to remove the ambiguity.

When more than one image is captured, among which at least one alert is raised, different rationales can be used depending upon the alert type. For example, an aggregated alert can be raised if a combination of potential anomalies are detected in the same image, or a group of successive images in the same group. For example, an aggregated alert can be raised if an anomaly A and an anomaly B are found in the same image, or in a group of successive images from the same medical device.

The aggregated alerts can also take into account both the alerts/potential anomalies, and anatomical structures that are detected. For example, in an example where alerts relative to stomach and UV are considered:
o If at least one image comprises a stomach and an alert, an aggregate alert is raised
o Otherwise, if at least one image with stomach and without alert has been captured, no aggregate alert is raised ;
o Otherwise (if no image of stomach has been captured), if at least one image with UV and alert has been captured, an aggregate alert is raised ;
o Otherwise (if no image of stomach, and no image with UV and alert has been captured), a notification to recommend to capture further images can be raised, in order to remove the ambiguity.

It is thus apparent that many different combinations of alerts can be considered, to highlight different levels of criticality of alerts, likelihoods of the alert being a true or a false positive, and importance of alerts.

The alert, or aggregate alert, rendered, or more generally the highlight of images based upon aggregated conditions at step S2.5 serves as a notification to the practitioner, providing them with important information that may require further examination or intervention. The alert is designed to be non-diagnostic, serving only to raise suspicion and prompt further investigation by the healthcare professional. This step S2.5 ensures that practitioners are promptly informed of potential issues.

The method P2 thus allows for an effective detection of potential issue, and alerting of practitioners.

As explained above, the method P2 can be executed on at least one computing device, such as the second computing device Dev2.1, in relation to at least one man-machine interface, for example carried on the tablet Tab1. The at least one man-machine interface serves to, and display, and when applicable highlight the medical image to the healthcare professional. In a number of embodiments, the at least one computing device, and the at least one man machine interface interact, so that the healthcare practitioner can provide inputs to the at least one computing device, for example to input the at least one region of interest at step S2.1.

Although the figure 1 represents one computing device Dev2.1, and one tablet Tab1 to carry on the at least one man machine interface, this architecture is provided by means of non limitative embodiment only. Other embodiments are foreseeable. For example:
- the steps of the method P2 may be computed on more than one computing device, for example through distributed computing ;
- the at least one man-machine interface could also be distributed across more than one device. The devices that carry on the man machine interface are not limited to tablets, and may for example comprise personal computers, smartphones, smartwatches, etc.
- the at least one man machine interface can also be carried on by a computing device that performs the image analysis. For example, the whole method could be executed by a tablet or a personal computer;
- all the steps of the method P2 can be executed on a single computing device, which captures ,analyzes and displays the medical image, and when applicable highlights it.

A plurality of examples of conditions that triggers the highlight of a medical images will now be presented.

In general, the images are highlighted when anomalies are suspected based upon the content of the images; However, the conditions for highlighting images can also be triggered because of an incorrect image captured. Certain potential anomalies can be suspected directly depending on the view defined by the ROI, and the anatomical structures that are detected, or conversely that are absent. Indeed, it is expected that certain anatomical structures, or combinations of anatomical structures, should be present for a given view. Medical images can thus highlightedwhen a ROI defining a view is obtained, but an unexpected combination of anatomical structures is detected.

For example, a potential great vessels malposition can be suspected, and the image highlighted if the view defined by the ROI is a Left Ventricular Outflow Tract "LVOT" view, and the detected anatomical structures comprise at least one of the combinations listed below:
- a left ventricle and a pulmonary artery trunk ;
- a left ventricle and a pulmonary artery bifurcation ;
- a pulmonary artery trunk and ascending aorta and a left ventricle ;
- a right ventricle and an ascending aorta ;
- a pulmonary artery bifurcation but no ascending aorta.

In another example, an absence of cavum septum pellucidum can be suspected, and the image highlighted if the view defined by the ROI is a transthalamic view and the detected anatomical structures does not comprise a cavum septum pellucidum.

In yet another example, an absence of corpus callosum can be suspected, and the image highlighted if the view defined by the ROI is a corpus callosum view and the detected anatomical structures does not comprise a corpus callosum.

It is now referred to figure 5.

Figure 5 shows a flowchart illustrating a method P5 for providing decision support in medical imaging, specifically aimed at highlighting medical images that require specific care for example to detect potential anomalies in medical images4.

The method P5 comprises all the steps S2.1 to S2.5 discussed with reference to figure 2, and the step S2.4 comprises all the subs-steps S5.1 to S5.4.

Step S5.1 introduces a condition related to the combination of the ROI and the anatomical structures detected at step S2.3. This condition acts as a trigger for the subsequent steps, ensuring that the segmentation and analysis are only performed when certain criteria are met. The condition may be defined according to the anomaly that is sought after. Few examples of such conditions and related analysis / anomalies will be presented hereinafter.

It is worth noting that, although the figure 5 is presented with a single condition at step S5.1, the invention is not restricted to the evaluation of a single condition. Quite to the contrary, a plurality of conditions, each associated with a suspicion of anomaly anomaly and a specific analysis of segmentation may be used. Stated otherwise, a plurality of conditions or highlighting medical images each associated with a possible anomaly and a specific analysis of segmentation may be assessed, and the steps S5.2 to S5.4 using rules and analysis specifically tailored for a potential anomaly may be executed each time a condition for further analysis is fulfilled.

If the condition (or none of the conditions is fulfilled at step S5.1, the method can return to step S2.1 to highlight medical images on the ground of further ROIs and/or further images.

If the condition is fulfilled at step S5.1, the process proceeds to step S5.2, which involves performing a segmentation of the detected anatomical structures within the ROI.

It is worth noting that, although the method P5 is described in an embodiment relative to the highlighting of medical images, in particular when anomalies are suspected in an image, the invention is not restricted to this example, and the segmentation can be used for other purposes. For example, the segmentation of structures can be simply displayed to a practitioners to improve the understanding of the medical images by the practitioner. In general, the improved segmentation of structures in a medical image is one of the objectives of certain embodiments.

The segmentation separates the pixels that belong to the anatomical structures from the other pixels of the image. Contrarily to the detection of anatomical structure at step S2.2 which may return a large region such as a bounding box encompassing the anatomical structure, the segmentation precisely identifies a distinct region that defines the anatomical structure at a pixel level.

Stated otherwise, the at least one segmented structure is a set of pixel, for example in the form of a pixel mask, which is deemed to contain all the pixels that belong to the anatomical structure, and only pixels that belong to the anatomical structure, thereby allowing to separate the anatomical structure from the rest of the image.

Several segmentation methods can be employed to perform the segmentation of anatomical structures in medical images. These methods include:
- Region-Based Segmentation: This method involves partitioning an image into regions based on predefined criteria, such as intensity or texture. Techniques like region growing and watershed algorithms fall under this category ;
- Edge-Based Segmentation: This approach detects edges within an image to define boundaries of anatomical structures. It often uses edge detection algorithms like the Canny or Sobel operators ;
- Clustering Methods: Techniques such as k-means clustering or Gaussian Mixture Models (GMM) group pixels into clusters based on their features, which can be used to segment different structures.
- Active Contour Models (Snakes): These models use curves that move through the spatial domain of an image to find object boundaries, driven by image forces and constraints ;
- Graph-Based Segmentation: This method models the image as a graph and uses algorithms like graph cuts to partition the image into segments ;
- Deep Learning-Based Segmentation: Convolutional neural networks (CNNs), such as U-Net or its variations like U2-Net, are widely used for semantic segmentation in medical imaging. These models are trained on labeled datasets to automatically learn and identify the boundaries of anatomical structures. For example, a CNN can be used to segment a specific type of anatomical structure ;
- Hybrid Methods: Combining multiple segmentation techniques can enhance accuracy and robustness, leveraging the strengths of each method to improve overall performance.

The choice of segmentation method depends on the specific requirements of the task, the quality of the images, and the computational resources available. By means of non-limitative example, an example of segmentation method based on a CNN will be presented with reference to figure 6.

In step S5.3, an analysis of the at least one segmented structure is conducted. This analysis evaluates the segmented structures to identify any irregularities or deviations from the norm that may indicate a potential anomaly. The insights gained from this analysis are important for the subsequent detection step. The analysis of the segmented structure may comprise computing different metrics from the segmented structure, such as for example their sizes, surface, angles, etc.

Step S5.4 involves detecting the presence or absence of at least one potential anomaly in the medical image, based on the output of the determination of anatomical structures and the analysis of their segmentation.

The analysis of the at least one segmented structure thus allows detecting the presence or absence of anomalies by determining a deviation from a norm of certain features of the anatomical structures. The combination of the determination of ROI, anatomical structures, and analysis of the segmentation of the anatomical structures therefore provides an accurate combination for detecting the potential anomalies. Few examples of determination of anomalies based on the detection of anatomical structures, and analysis of the segmentation of the anatomical structures will be provided below.

As for the method P2:
- If no image to highlight is detected at step S5.4, the method can return to step S2.1 to images to highlight on the ground of further ROIs and/or further images;
- if at least one the medical image is detected to highlight, the step S5.4 is activated, where the medical image is highlighted through at least one man-machine interface.

It is now referred to figure 6.

Figure 6 is an example of detection and segmentation of anatomical structures and according to an embodiment using a segmentation model associated with a specific view.

The process begins with a medical image, noted IMG6, which serves as the input for the detection of the anatomical structure, and one of the inputs of the segmentation model.

In the example of figure 6, the step S2.2 detected 3 anatomical structures, represented respectively by three bounding boxes, namely Bbox6.1, Bbox6.2, and Bbox6.3, each corresponding to distinct anatomical structures within the image. Each bounding box represents a rectangular region in the mage IMG6 that encompasses an anatomical structure.

A segmentation model associated to the view defined by the ROI obtained at step S2.1, which can be for convenience noted SEGMENT_STRUCUTRES, is retrieved. Stated otherwise, a specific view (for example a LVOT view, a 3 vessels view, etc.) is associated with a segmentation model specifically trained for that view, thus enabling the model to process the input data with precision.

The model SEGMENT_STRUCTURES receives as input the bounding boxes associated with the detected anatomical structures, and the entire medical image on separate channels. In the example of the figure 6, the segmentation model SEGMENT_STRUCTURES receives 4 inputs on 4 different channels:
- the first bounding box Bbox6.1 corresponding to the first detected anatomical structure ;
- the second bounding box Bbox6.2 corresponding to the second detected anatomical structure ;
- the third bounding box Bbox6.3 corresponding to the third detected anatomical structure ;
- the entire image IMG6.

This multi-channel input approach improves the model's capability to precisely segment the anatomical structures, as the model can utilize the comprehensive context of the image along with the focused regions defined by the bounding boxes. Furthermore, using a segmentation model associated to the view (which is itself associated with certain anatomical structures) and specifically trained for that view means that the model will be trained to take as input, and segment the specific anatomical associated to the view.

The model SEGMENT_STRUCTURE may for example be a fully convolutional network that comprises one input channel for the medical image, and one input channel for each of the anatomical structures associated with the view. By means of non-limitative example, the model SEGEMNT_STRUCTURE may be a U²-Net model described by Qin, X., Zhang, Z., Huang, C., Dehghan, M., Zaiane, O. R., & Jagersand, M. (2020). U2-Net: Going deeper with nested U-structure for salient object detection. Pattern recognition, 106, 107404.

The architecture of the U²-Net model can thus be adapted to match the number of anatomical structures associated to a given view. For example, if a view is associated to 3 anatomical structures, the number of input and output channels of the U²-Net model is set to 3, to use as input the 3 bounding boxes of the anatomical structures, and as output the 3 segmentation masks. In a PyTorch implementation of U²-Net, this involves adjusting in_channels in the first layer and out_channels in the last layer accordingly so that the modified U²-Net can process multi-channel data. As noted above, the U²-Net model is provided by means of non limitative example only of a SEGMENT_STRUCTURE model. The modification of the number of channels can more generally be applied to any kind of multi-channel CNN, or even more generally any kind of multi-channel segmentation model.

The SEGMENT_STRUCTURE model has been previously trained to segment the anatomical structures. In particular, wherein each training sample comprises a medical image, and the bounding boxes output by the DETECT_STRUCTURE model can be manually annotated to associate each structure with a segmentation mask. Then the SEGMENT_STRUCTURE model can be trained to segment the structures. During training, techniques such as data augmentation (e.g., rotation, flipping, scaling) can be applied to increase the diversity of the training data and improve model robustness.

As explained above, a different model DETECT_STRUCTURE is used for each view where a segmentation is used. Therefore, the training database of the model DETECT_STRUCTURE contains only images of the view to which it is associated.

Certain pre-processing steps may be performed between the detection of anatomical structures and their segmentation. For example:
- the image IMG6 can be converted from RGB format to grayscale to reduce the dimension. This enhances the accuracy of the segmentation, because a CNN specifically trained to segment the structure of a given view is particularly efficient to generate segmentation masks based on grayscale images ;
- background removal can performed using the output of the DETECT_STRUCTURE model. For example, the pixels outside the bonding boxes encompassing the detected structures can be set to 0,
- the image IMG6 can be resized to a specific dimension that is expected as input to the segmentation model SEGMENT_STRUCTURE, for example to a 840x1120 dimension.

Upon processing the input, the segmentation model outputs segmentation masks, SegMsk6.1, SegMsk6.2, and SegMsk6.3, each respectively corresponding to the anatomical structures defined by the bounding boxes Bbox6.1, Bbox6.2, and Bbox6.3. These segmentation masks provide a pixel-level delineation of the structures, offering a precise representation of their boundaries within the medical image.

In an embodiment of the invention, a 5-fold cross-validation approach is employed to ensure the segmentation process's robustness. The final segmentation masks are generated through a majority voting mechanism applied to the predictions for each pixel: a pixel is classified as belonging to the structure if at least three out of five models concurred, and vice versa. In embodiments wherein such a majority voting is used, 5 different instances of the same model SEGMENT_STRUCTURE are trained from 5 different random initialization respectively and different training sets, in order to obtain, for a given view not a single SEGMENT_STRUCTURE, but 5 SEGMENT_STRUCTURE models. Then the 5 SEGMENT_STRUCTURE models can be used for cross-validation. The different training sets may be different parts of a global training set comprising intersections (i.e, certain training samples of the initial training set may belong to a plurality of training sets of the 5 models but at least some of the training samples are distinct between the models).

The segmentation masks SegMsk6.1, SegMsk6.2, and SegMsk6.3 thus provide an input for the step S5.3 of analyzing the at least one segmented structure. Indeed, a segmentation mask allows computing various features of a segmented structure, such as for example its sizes, angles surface, etc.

Although the segmented structures are represented in figure 6 as segmentation masks, the invention is not limited to this representation, and other representations of segmented structures may be considered, provided that they allow a pixel-wise definition of an anatomical structure in the medical image.

In the example of figure 6, a final output, SegIMG6, is the medical image with the segmentation masks overlaid, highlighting the segmented anatomical structures. This overlay provides a clear visual representation of the structures, facilitating further analysis and interpretation by healthcare professionals. The provision of such medical image with segmentation masks overlaid provides a support to healthcare professional for anomaly detection. Such a visual output is however not always provided, as the detection of possible anomalies will primarily rely, in embodiments where segmentation is performed, on an automatic analysis of the segmented structures.

The technical effect of using a model specifically trained for the view is the provision of an accurate segmentation of the structures within that view. By including the complete image in addition to the bounding boxes, the model can retrieve data from the structures that might not be fully contained within the bounding boxes, thus enhancing the accuracy of the segmentation. This approach ensures that the segmentation method is robust and reliable, providing meaningful insights into the anatomical structures present in the medical image.

Now certain examples of detections that an image is to highlight will be described.

As explained above the method P5 relies on a double condition:
- at step S5.1, a condition relative to the combination of at least one region of interest and said at least one anatomical structure in the region of interest, is evaluated, to determined whether to perform or not the segmentation of anatomical structures ;
- at step S5.4, a detection that the image is to highlight based upon the output of the determination of anatomical structures and said analysis of said least one segmented structure.

In each example, we will thus present how the two steps S5.1 and S5.4 are performed, as well as the analysis at step S5.3.

In a number of embodiments relative to potential anomalies relative to inconsistent widths of the 3 vessels, the detection has the following specificities:
- the view if a 3 vessels view ;
- the structures to segment comprise a pulmonary artery, an ascending aorta and a superior vena cava ;
- therefore, the condition at step S5.1 is validated if the view if a 3 vessels view, and the at least one anatomical structure comprises a pulmonary artery, an ascending aorta and a superior vena cava ;
- at step S5.2, the pulmonary artery, ascending aorta and superior vena cava are segmented. As explained above, a specific model trained on 3 vessels view can be used to segment simultaneously the pulmonary artery, ascending aorta and superior vena cava structures ;
- at step S5.3, the analysis of the segmented structures comprises a computation of a width of the pulmonary artery, a width of the ascending aorta and a width of the superior vena cava.

The segmentation of the pulmonary artery, ascending aorta and superior vena cava structures allows determining, for example on segmentation masks, a width of the pulmonary artery, a width of the ascending aorta and a width of the superior vena cava.

The computation of the widths of the three structures highlighting a medical image in case of inconsistent widths of the 3 vessels, which may be representative of a fetal anomaly.

In a number of embodiments of the invention:
- the step S5.3 comprises calculating a ratio between the width of the pulmonary artery, and the width of the aorta ;
- the step S5.4 comprises detecting that the medical image is to highlight, if the ratio between the width of the pulmonary artery, and the width of the aorta is above a predefined threshold (of ratio between the width of the pulmonary artery, and the width of the aorta).

The use of the ratio between the width of the pulmonary artery, and the width of the aorta is above a predefined threshold allows automatically highlighting a medical image that represents a potential an asymmetry of the great artery.

The determination of the predefined threshold can have been performed in many different ways, for example be determined by:
- considering a large number of clinical cases ;
- calculating ratios between the width of the pulmonary artery, in and the width of the aorta ;
- correlating the calculated ratios with clinical outcomes or diagnoses, i.e attributing to each ration a "normal" or "abnormal" label ;
- determining a threshold that distinguishes between normal and abnormal cases. This threshold can for example be set at a value that maximizes sensitivity and specificity for detecting a particular condition. For example, a threshold might be set at a value where the majority of normal cases fall below the threshold, while abnormal cases fall above it.

The examples are provided by means of non limitative examples only of the determination of a predefined threshold of ratio between the width of the pulmonary artery, and the width of the aorta that allows distinguishing potential anomalies. It is apparent that any method allowing to determined such threshold based upon a preliminary study of clinical cases could be used.

The above considerations can be extended to any predefined threshold or range used within an embodiment of the invention.

In a number of embodiments of the invention:
- the step S5.3 comprises calculating between the width of the aorta, and the width of the superior vena cava;
- the step S5.4 comprises detecting that the medical image is to highlight, if the ratio between the width of the aorta, and the width of the superior vena cava is above a predefined threshold (of ratio between width of the aorta, and width of the superior vena cava).

The use of the ratio between the width of the aorta, and the width of the superior vena cava is above a predefined threshold allows automatically highligthing an image that represents a potential an hypoplasia of the ascending aorta.

In a number of embodiments relative to highlighting images that represent potential anomalies relative to disequilibrium of the chambers, the detection has the following specificities:
- the view if a 4 chamber view ;
- the structures to segment comprise a left ventricle, a right ventricle, a left atrium, and a right atrium ;
- therefore, the condition at step S5.1 is validated if the view if a 4 chamber view, and the at least one anatomical structure comprises a left ventricle, a right ventricle, a left atrium, and a right atrium ;
- at step S5.2, the left ventricle, right ventricle, left atrium, and right atrium are segmented. As explained above, a specific model trained on 4 chamber view can be used to segment simultaneously the left ventricle, right ventricle, left atrium, and right atrium structures, using 4 channels for the 4 structures respectively ;
- at step S5.3, the analysis of the segmented structures comprises a computation of a width of the left ventricle, a width of the right ventricle, a width of the left atrium, and a width of the right atrium ;
- the step S5.4 comprises highlighting the medical image if at least one or the conditions listed below is fulfilled:
   o a ratio between the width of left atrium and the width of the right atrium is not in a first predefined range ; or
   o a ratio between the width of left ventricle and the width of the right ventricle is not in a second predefined range.

Stated otherwise,:
- a first predefined range of "expected" ratios between the width of left atrium and the width of the right atrium is predefined, and the image is detected to highlight if the ratio between the width of left atrium and the width of the right atrium is not in first predefined range ;
- a second predefined range of "expected" ratios between width of left ventricle and the width of the right ventricle is predefined, the image is detected to highlight if the ratio between the width of left ventricle and the width of the right ventricle is not in the second predefined range.

The verification that the ratios fall in the first, respectively second predefine ranges, allows highlighting a medical image that represents a potential disequilibrium of the chambers.

In a number of embodiments relative to highlighting images that represent potential anomalies relative to large heart, the detection has the following specificities:
- the view if a 4 chamber view ;
- the structures to segment comprise a left ventricle, a right ventricle, a left atrium, a right atrium, and a thorax contour ;
- therefore, the condition at step S5.1 is validated if the view if a 4 chamber view, and the at least one anatomical structure comprises a left ventricle, a right ventricle, a left atrium, a right atrium, and a thorax contour;
- at step S5.2, the left ventricle, right ventricle, left atrium, right atrium and thorax contour are segmented. As explained above, a specific model trained on 4 chamber view can be used to segment simultaneously the left ventricle, right ventricle, left atrium, right atrium, and thorax contour structures, using 5 channels for the 5 structures respectively ;
- at step S5.3, the analysis of the segmented structures comprises:
   o a determination of a surface of the heart in the medical image as the sum of the areas of segmentation masks of the left ventricle, the right ventricle, the left atrium, and the right atrium ;
   ∘ a determination of a surface of the abdomen in the medical image as the area of the segmentation mask of the thorax contour ;
   ∘ a calculation of a ratio between the surface of the heart and the surface of the abdomen ;
- the step S5.4 comprises highlighting said medical image if said ratio (between the surface of the heart and the surface of the abdomen) is above a predefined threshold of ratio between the surface of the heart and the surface of the abdomen).

Stated otherwise:
- the surface of the heart is determined as the sum of the surfaces of the left ventricle, the right ventricle, the left atrium, and the right atrium, each of the surfaces of the anatomical structures being calculated as the area of the corresponding segmentation mask ;
- the surface of the abdomen is determined as the area of the segmentation mask of the thorax contour ;
- the image is highlighted if the surface of the heart is considered as "too large" compared to the surface of the abdomen, i.e if the ration between the surface of the heart and the surface of the abdomen is above the predefined threshold.

Highlighting a medical image if said ratio (between the surface of the heart and the surface of the abdomen) is above a predefined threshold of ratio between the surface of the heart and the surface of the abdomen allows highlighting an image that represents a potential an abnormally large heart.

It is now referred to figure 7.

Figure 7 is an example of detection of potential deviation of the heart axis

In a number of embodiments relative to highlighting medical images that represent potential anomalies relative to deviation of the heart axis, the detection has the following specificities:
- the view if a 4 chamber view, defined in the example of figure 7 by the region of interest ROI7 ;
- the structures to segment comprise an interventricular septum, a spine, a left ventricle, a right ventricle, a left atrium, a right atrium and a thorax contour;
- therefore, the condition at step S5.1 is validated if the view if a 4 chamber view, and the at least one anatomical structure comprises an interventricular septum, a spine, a left ventricle, a right ventricle, a left atrium, a right atrium and a thorax contour ;
- at step S5.2, the interventricular septum, spine, left ventricle, right ventricle, left atrium, right atrium and thorax contour are segmented. As explained above, a specific model trained on 4 chamber view can be used to segment simultaneously the 7 structures, using 7 channels for the 7 structures respectively ;
- at step S5.3, the analysis of the segmented structures comprises:
   o a determination of a first line, noted Lin7.1, going through the septum. In particular, as the septum comprises the tip of the heart, the line Lin7.1 may indicate the direction to which is directed the heart. The first line Lin7.1 can be obtained by fitting a linear regression on the mask of the interventricular septum;
   o a determination of a second line, noted Lin7.2, that contains the center of a bounding box enclosing the spine and divides the thorax contour into two parts of equal surface;
   ∘ a calculation of an angle, noted Ang7 between the first line and the second line;
   the step S5.4 comprises detecting that the medical image is to highlight if said angle is not in a predefined range.

Stated otherwise:
- The two lines Lin7.1 and Lin7.2 allow calculating the angle Ang7 ;
- the image is determined to highlight, if the angle falls outside of a predefined range corresponding to the "expected" angles.

As the septum goes to the tip of the heart so it gives the "direction" towards which the heart points. The spine, is centered since the thorax is supposed to be symmetrical so the second line Lin7.2 corresponds to the line which separates the thorax in two and the angle with the septum, represented by the first line Lin7.1, therefore indicates how far the heart points towards the extremities or rather towards the center.

Detecting a potential deviation of the heart axis if said angle is not in a predefined range allows highlighting to a user an image that represented a potential a deviation of the heart axis.

The detections listed above are provided by means of non limitative examples only of detections of images to highlight according to a number of embodiments. It is worth noting that other potential anomalies may be determined.

In addition, a plurality of potential anomalies could be detected simultaneously, by applying, sequentially or in parallel, different embodiments corresponding to different anomalies.

This disclosure is not limited to the device, method and computer program described here, which are only examples. The invention encompasses every alternative that a person skilled in the art would envisage when reading this text..

## Claims

1. A computer-implemented method (P2; P5) comprising:
- obtaining (S2.1) at least one region of interest "ROI" (ROI3; ROI7) defining a view in a medical image (IMG3; IMG4; IMG6) ;
- detecting (S2.2) at least one anatomical structure in the region of interest ;
- detecting (S2.3) a combination of said at least one region of interest and said at least one anatomical structure in the region of interest.

2. The computer-implemented method of claim 1, wherein:
- detecting at least one anatomical structure in the region of interest and detecting a combination of said at least one region of interest and said at least one anatomical structure in the region of interest are performed by at least one computing device;
- obtaining at least one region of interest defining a view in a medical image comprises receiving, by said at least one computing device, said medical image previously captured by a medical device physically distinct from said at least one computing device.

3. The method of any one of the preceding claims, further comprising:
- detecting (S2.4) that said medical image is to highlight based at least upon said detected combination
- if it is detected that said medical is to highlight , highlighting (S2.5) said medical image through at least one man interface.

4. The method of claim 3, comprising highlighting said medical image upon the occurrence of at least one condition relative to the detection that said medical image is to highlight, and at least one of :
- the detection that at least one further medical image captured by the same medical device than said medical image is to highlight ;
- the detection of a presence or absence of at least one further condition of highlight said medical image, or in at least one further medical image captured by the same medical device than said medical image ;
- the detection of a presence or absence of at least one further anatomical structure in said medical image, or in at least one further medical image captured by the same medical device than said medical image.

5. The method of claim 3, comprising detecting that said medical image is to highlight if:
- said view is a Left Ventricular Outflow Tract "LVOT" ;
- said at least one anatomical structure comprises at least one of:
∘ a left ventricle and a pulmonary artery trunk ;
∘ a left ventricle and a pulmonary artery bifurcation ;
∘ a pulmonary artery trunk and ascending aorta and a left ventricle ;
∘ a right ventricle and an ascending aorta ;
∘ a pulmonary artery bifurcation but no ascending aorta.

6. The method of claim 3, comprising detecting that said medical image is to highlight if:
- said view is a transthalamic view ;
- said at least one anatomical structure does not comprise a cavum septum pellucidum.

7. The method of claim 3, comprising detecting that said medical image is to highlight if:
- said view is a corpus callosum view ;
- said at least one anatomical structure does not comprise a corpus callosum.

8. The method (P5) of any of claims 1 to 2, further comprising, upon a condition relative to said combination (S5.1):
- performing a segmentation (S5.2) of said at least one anatomical structure in the region of interest of the image.

9. The method of claim 8, wherein said performing the segmentation comprises:
- retrieving a segmentation model associated to said view ;
- inputting to said segmentation model, on separate channels :
o at least one bounding box (Bbox4.1; Bbox4.2, BBox4.3) corresponding respectively to each of said at least one anatomical structure ;
∘ said medical image ;
- receiving, as output from the segmentation model, at least one structure segmentation mask (SegMsk4.1; SegMsk 4.2, SegMsk 4.3) corresponding respectively to each of said at least one anatomical structure.

10. The method of claim 9, wherein:
- the segmentation model comprises a fully convolutional network that comprises one input channel for the medical image, and one input channel for each of the anatomical structures associated with the view ;
- said inputting comprises:
∘ converting said medical image, and each of said at least one bounding box into grayscale images ;
∘ Inputting said grayscale images to said segmentation model.

11. The method of any one of claims 8 to 10, further comprising:
- performing an analysis (S5.3) of the at least one segmented structure
- detecting (S5.4) that said medical image is to highlight based upon the output of said determination of anatomical structures and said analysis of said at least one segmented structure.

12. The computer-implemented method of claim 11, wherein:
- said condition relative to the combination is validated if:
∘ said view is a 3 vessels view, and
∘ said at least one anatomical structure comprises a pulmonary artery, an ascending aorta and a superior vena cava ;
- said analysis of the at least one segmented structure comprises a computation of a width of the pulmonary artery, a width of the ascending aorta and a width of the superior vena cava.

13. The computer-implemented method of claim 12, comprising detecting that said medical image is to highlight, if a ratio between the width of the pulmonary artery, and the width of the aorta is above a predefined threshold.

14. The computer-implemented method of claim 11, wherein:
- said condition relative to the combination is validated if:
∘ said view is a 4 chamber view, and
∘ said at least one anatomical structure comprises a left ventricle, a right ventricle, a left atrium, and a right atrium ;
- said analysis of the at least one segmented structure comprises a computation of a width of the left ventricle, a width of the right ventricle, a width of the left atrium, and a width of the right atrium;
said method comprising detecting that said medical image is to highlight if:
- a ratio between the width of left atrium and the width of the right atrium is not in a first predefined range; or
- a ratio between the width of left ventricle and the width of the right ventricle is not in a second predefined range.

15. A computing system (Sys1) comprising at least one processing unit and configured to execute a method according to one of claims 1 to 14.

16. Computer software comprising instructions to implement at least a part of a method according to one of claims 1 to 14 when the software is executed by a processor.
